# EUROPEAN PATENT APPLICATION

(11) **EP 4 832 001 A1**
(43) Date of publication of application: **28.10.2026**
(21) Application number: 25171824.3
(22) Date of filing: 22.04.2025
(51) Int. Cl.: G16H 30/20, G16H 30/40, G16H 50/20

(54) **METHOD FOR THE GENERATION OF SYNTHETIC 3D ACTIVITY DISTRIBUTION MAPS**

(71) Applicant: Universität Bern, 3012 Bern (CH)
(72) Inventor: Lopez Montes, Alejandro, 3010 Bern (CH); Shi, Kuangyu, 3010 Bern (CH); Rominger, Axel, 3010 Bern (CH); Afshar-Oromieh, Ali, 3010 Bern (CH)
(74) Representative: Longchamp, Jean-Nicolas

(57) **Abstract**

The described method pertains to generating synthetic three-dimensional (3D) activity distribution maps in nuclear medicine imaging, addressing the limitations of planar scintigraphy images. This method utilizes a generative Artificial Intelligence (AI) model trained on 3D nuclear medicine patient specific datasets, such as PET or SPECT images, to transform one or two planar scintigraphy images into a synthetic 3D activity distribution map. This approach enhances the precision and utility of nuclear medicine imaging by providing detailed 3D maps without the need for costly SPECT imaging. The use of patient-specific datasets ensures accurate representation of individual anatomy and radionuclide distribution. This method is particularly useful for post-radionuclide therapy dosimetry and treatment effectiveness studies, improving clinical applications in theranostics. The approach allows centers without specialized volumetric imaging equipment to produce high-resolution 3D maps, optimizing treatment planning and patient outcomes.

## Description

### Technical Field

The present disclosure pertains to the domain of nuclear medicine imaging, particularly focusing on techniques for creating three-dimensional activity distribution maps derived from planar scintigraphy images through the application of artificial intelligence methodologies leveraging previous data of the patient.

### Background of the invention

Theranostics has experienced an increased interest during the last years. Targeted treatments for oncologic patients enabled by specific radiotracers for malignancies such as thyroids or prostate tumors and their metastasis are undergoing rapid growth. The radiopharmaceutical ¹⁷⁷Lu-PSMA-617 (with the radionuclide ¹⁷⁷Lu) for the treatment of prostate cancer and ¹⁷⁷Lu-Dotatate for the treatment of neuroendocrine tumor are among the most promising and currently routinely used cases of nuclear medicine (NM) for cancer treatment with application in theranostics. The additional gamma rays emitted by the radionuclide ¹⁷⁷Lu can be used to achieve an image of the actual biodistribution of the drug, allowing dosimetry and treatment effectiveness studies.

2D-planar imaging, also called scintigraphy in some modalities, is a common technique used in theranostics for monitorization of the drug distribution. It generally consists in the acquisition of, conventionally, two (frontal and posterior) full body planar acquisitions of the gamma rays emitted by radionuclides with the use of gamma cameras. However, the dosimetry and radionuclide distribution information given by planar images is limited since they can only provide 2D distribution of the radionuclide which dampens their usability in clinical practice as a quantitative method. The alternative 3D imaging used is generally SPECT imaging after the treatment to locate the distribution of the molecule in the organism. However, SPECT is generally a costly and slow procedure, needing multiple bed positions to acquire full body images of the patient and requires acquisitions at different angles which can lead to very long acquisitions of up to 1h for full body SPECT studies. Furthermore, SPECT image quality is suboptimal when compared with PET imaging. Some works have explored the use of fast SPECT protocols reducing either the time per view or the number of views (sparse SPECT) With all, SPECT still results in increased costs for the hospitals and a dedication of resources (expensive scanners) and personnel (e.g. working hours for technicians).

There is thus a need for a method that allows obtaining more accurate post-radionuclide therapy dosimetry and radionuclide distribution information while avoiding the drawbacks associated with post-radionuclide therapy SPECT imaging. Such a method would enhance clinical applications in theranostic in a more efficient, cost-effective, and high-resolution manner.

### Summary of the invention

The object of the present invention is to propose novel computer-implemented methods, with which the above-described drawbacks of the known methods are completely overcome or at least greatly diminished.

According to a first aspect, the present invention relates to a computer-implemented method for generating a synthetic post-radionuclide therapy three-dimensional (3D) activity distribution map of a patient comprising:
a. Obtaining at least one, advantageously two, post-radionuclide therapy planar scintigraphy images of the patient;
b. Providing a generative Artificial Intelligence (AI) model trained on a patient-specific 3D medical imaging dataset of radiotracer distribution derived from at least one pre-therapy image of the patient, preferably from a nuclear medicine technique such as a Positron Emission Tomography (PET) or Single Photon Computed Tomography (SPECT), or alternatively derived from an anatomic image technique such as Computed Tomography (CT), to generate from one, advantageously two, planar images a synthetic 3D activity distribution map of the radionuclide;
c. Employing the obtained planar scintigraphy images as input or sampling constraint for said generative Al model to generate a synthetic post-radionuclide therapy 3D activity distribution map of the patient.

The technical effects associated with the described computer-implemented method for generating synthetic post-radionuclide therapy three-dimensional activity distribution maps include the ability to transform limited planar scintigraphy images into comprehensive 3D activity maps, thereby enhancing the precision and utility of nuclear medicine imaging, while avoiding costly and resource intensive post-radionuclide therapy SPECT imaging. By leveraging a generative Al model trained on a patient specific dataset of 3D medical imaging dataset of radiotracer distribution derived from at least one pre-therapy image of the patient, preferably from a nuclear medicine technique such as a Positron Emission Tomography (PET) or Single Photon Computed Tomography (SPECT), or alternatively derived from an anatomic image technique such as Computed Tomography (CT), the method effectively bridges the gap between planar and volumetric imaging. This approach allows for the generation of detailed synthetic 3D maps from minimal input data, specifically one or two planar images, which are typically more accessible, less expensive and quicker to acquire than full SPECT volumetric scans. The training of the Al model using patient-specific datasets ensures that the generated 3D maps accurately reflect individual patient anatomy and radionuclide distribution, leading to improved dosimetry evaluations and diagnostic assessments. Additionally, employing the obtained planar scintigraphy images as input or constraints for the Al model ensures that the synthetic 3D maps are consistent with the actual post-radionuclide therapy imaging data, thereby enhancing the reliability and clinical relevance of the generated maps.

Overall, the method provides a significant advancement in nuclear medicine imaging by enabling centers without specialized SPECT volumetric imaging equipment to produce detailed 3D activity maps, thus improving treatment planning, monitoring, and patient outcomes. This allows also for a reduction of the resources associated for post-radionuclide therapy imaging, as no SPECT imaging is necessary and only faster and less expensive scintigraphy imaging is required. Currently, the most used technic for the diagnosis of prostate cancer is PET imaging using for instance ¹⁸F-PSMA or ⁶⁸Ga-PSMA as labelling agents. Thus, most patients undergoing radiopharmaceutical therapy, as for example ¹⁷⁷Lu-PSMA therapy, count anyway with a pre-treatment PET/CT study. The present invention builds on this aspect and allows for the generation of post-radionuclide therapy 3D distribution maps based on the information anyway at hand for the diagnosis of the patient and readily available post-radionuclide therapy scintigraphy imaging.

In a first preferred embodiment of the first aspect of the present invention, the patient-specific 3D medical imaging dataset of radiotracer distribution comprises or consists of simulated possible 3D uptake distribution maps of the radionuclide derived from the at least one pre-radionuclide therapy 3D medical image. By utilizing simulated 3D uptake distribution maps based on pre-radionuclide therapy images, the generative Al model can more effectively capture the unique anatomical and physiological characteristics of individual patients. This approach ensures that the synthetic 3D maps accurately reflect the patient's specific radionuclide distribution patterns, leading to improved dosimetric evaluations and diagnostic assessments.

In another preferred embodiment of the first aspect of the present invention, the model has been pre-trained on generic population datasets to generate 3D activity distributions from 2D planar images. This enables faster training for the patient-specific datasets and reduced computational resources, building the models on previously trained models on extensive datasets for generic population to generate 3D activity distributions from planar images are recommended, limiting the environmental and economical impact of training new datasets by starting the training from a problem specific optimized starting point.

In another embodiment of the first aspect of the present invention, the generative machine learning model is selected from the group consisting of Generative Adversarial Networks (GANs), Variational Autoencoders (VAE), Convolutional Neural Networks (CNN), and/or Diffusion Models (DM). By utilizing these advanced Al models, the invention can effectively capture complex spatial patterns and relationships inherent in nuclear medicine imaging data. Each model type offers unique strengths: GANs are adept at generating high-quality synthetic data, VAEs provide efficient encoding and decoding of complex data structures, CNNs excel in processing image data with spatial hierarchies, and Diffusion Models offer robust handling of noise and uncertainty in data and versatile and easy to train generative models with quality comparable to GANs. This versatility allows the method to be tailored to specific imaging requirements and datasets, ensuring high fidelity and accuracy in the generated 3D maps.

In a further preferred embodiment of the first aspect of the present invention, knowledge on the acquisition instrument and/or parameters used to acquire the planar scintigraphy images is included in the training, the refining and/or sampling of the model. This knowledge comprises advantageously the type of radionuclide, the energy of emitted photons, the collimator type, the energy window settings, the acquisition time and the distance between the detector and the patient. By integrating specific information about the imaging process, such as the type of radionuclide, photon energy, collimator type and properties, septa size, type of detector, pixel size of the detector, energy window settings, acquisition time, and detector-to-patient distance, the generative Al model can more precisely account for the nuances and variations inherent in the imaging data. This leads to a more accurate representation of the radionuclide distribution and patient anatomy in the synthetic 3D maps. Consequently, the inclusion of acquisition-specific knowledge enhances the model's ability to produce outputs that are consistent with the actual imaging conditions, thereby increasing the reliability and clinical applicability of the generated maps.

In another preferred embodiment of the first aspect of the present invention, anatomical and/or radionuclide distribution information corresponding to the 3D medical images, preferably PET/CT or SPECT/CT images, is included in the training and/or sampling of the model. By incorporating detailed anatomical and/or radionuclide distribution data, the generative Al model can more effectively capture the unique spatial characteristics, and biological variations present in individual patients. This results in synthetic 3D activity distribution maps that more accurately reflect the patient's specific anatomy and radionuclide distribution, leading to improved dosimetry evaluations and diagnostic assessments. The inclusion of such comprehensive data ensures that the generated maps are not only consistent with the actual imaging conditions but also tailored to the patient's unique physiological attributes, thereby increasing the clinical relevance and reliability of the imaging outputs.

In another preferred embodiment of the first aspect of the present invention, the anatomical information is obtained from Computed Tomography (CT), Ultrasounds (US) or Magnetic Resonance (MR) images scans. The anatomical maps can also be generated directly from the PET or SPECT scans via Al or using computer algorithms able to reconstruct activity and attenuation simultaneously. This allows the generative Al model to incorporate a comprehensive view of the patient's anatomy, which is crucial for accurately modeling radionuclide distribution. This integration allows the model to account for various anatomical structures and variations, leading to synthetic 3D activity distribution maps that more precisely reflect the patient's unique physiological characteristics. Consequently, this approach enhances the reliability and clinical relevance of the generated maps, improving dosimetry evaluations and diagnostic assessments. The use of anatomic images such as CT, US, or MR scans ensures that the synthetic maps are tailored to the patient's specific anatomical features, thereby contributing to more effective treatment planning and monitoring in nuclear medicine, ultimately leading to better patient outcomes.

In yet another preferred embodiment of the first aspect of the present invention, the generative machine learning model is a Convolutional Neural Network (CNN) model and said CNN model has been trained using a dataset comprising synthetic planar scintigraphy images derived from 3D PET images and the corresponding original 3D PET images serving as ground truth for training, such that the trained CNN model generates a 3D activity distribution map from one, advantageously two, input planar scintigraphy images. By training the CNN model with this comprehensive dataset, the model learns to effectively translate the limited information from planar scintigraphy images into detailed 3D activity maps. This training approach ensures that the CNN model captures the complex spatial relationships and patterns present in the 3D PET images, allowing it to generate synthetic maps that closely resemble the actual 3D activity distribution maps. As a result, the generated maps provide a more accurate representation of radionuclide distribution, improving dosimetry evaluations and diagnostic assessments. This embodiment enhances the clinical utility of planar scintigraphy by enabling the production of high-quality 3D maps without the need for specialized volumetric imaging equipment, thereby facilitating better treatment planning and monitoring in nuclear medicine. The CNN model is for instance a 3D ResUnet.

In a further preferred embodiment of the first aspect of the present invention, the generative machine learning model is based on Diffusion Model (DM) and the sampling process comprises guiding the model such that two-dimensional (2D) projection images generated from the 3D activity distribution map match the obtained post-radionuclide therapy planar scintigraphy image of the patient. By employing a diffusion model, for instance a MBIR model, the method effectively handles noise and uncertainty in the data, ensuring robust generation of 3D maps. The guided sampling process ensures that the intermediate 2D projections align with the actual planar scintigraphy images, thereby enforcing consistency between the synthetic 3D activity distribution maps and the real imaging data. This approach enhances the reliability and clinical relevance of the generated maps, as it ensures that the synthetic outputs are not only accurate but also reflective of the actual post-treatment conditions.

In another preferred embodiment of the first aspect of the present invention, guiding the DM comprises:
i. computing 2D projections from the intermediate 3D activity distribution maps generated during sampling and the anatomical information of the patient; and
ii. enforcing consistency between said 2D projections and the obtained post-radionuclide therapy planar scintigraphy images using a loss function, thereby steering the sampling process towards generating a 3D activity distribution map that is consistent with both the obtained scintigraphy images and the learned 3D activity distribution map.

By computing 2D projections from the intermediate 3D maps, the method ensures that the synthetic outputs are closely aligned with the actual planar scintigraphy images, thereby maintaining consistency between the generated maps and the real imaging data. The use of a loss function to enforce this consistency steers the sampling process towards producing 3D maps that accurately reflect both the obtained scintigraphy image and the learned 3D activity distribution map.

According to a second aspect, the present invention relates to a computer-implemented method for training a generative Al model to generate synthetic post-radionuclide therapy three-dimensional (3D) activity distribution maps of a patient, comprising:
a. Providing a patient-specific 3D medical imaging dataset of radiotracer distribution derived from at least one pre-radionuclide therapy 3D anatomical and/or radionuclide image of the patient, for instance a Positron Emission Tomography (PET), Single Photon Emission Tomography (SPECT), Computed Tomography (CT) and/or Magnetic Resonance (MR) image, representing possible post-radionuclide therapy uptake distribution maps;
b. Training the generative Al model using the patient-specific dataset to learn to generate 3D activity distribution maps from one, advantageously two, 2D images, such that the trained model is configured to generate a synthetic post-radionuclide therapy 3D activity distribution map of the patient using one, advantageously two, post-radionuclide therapy planar scintigraphy images as inputs or sampling constraints.

The technical effects associated with the second aspect of the present invention, which involves a computer-implemented method for training a generative Al model to generate synthetic post-radionuclide therapy three-dimensional activity distribution maps of a patient, include enhanced accuracy and personalization in the generation of 3D activity distribution maps, while avoiding the drawbacks associated with post-radionuclide therapy SPECT imaging. By providing a patient-specific 3D medical imaging dataset of radiotracer distribution derived from pre-radionuclide therapy 3D anatomical and/or radionuclide images, such as PET, SPECT, CT, or MR images, the Al model is trained to understand and generate 3D activity distribution maps from 2D images. This training process equips the model with a detailed understanding of the patient's unique anatomical and physiological attributes, ensuring that the synthetic maps accurately reflect individual patient characteristics. The use of patient-specific datasets allows the model to tailor its outputs to the specific attributes of each patient, thereby increasing the clinical relevance and reliability of the imaging results. Consequently, this approach enhances the precision of dosimetric evaluations and diagnostic assessments, facilitating more effective treatment planning and monitoring in nuclear medicine. Ultimately, the method contributes to better patient outcomes by providing detailed and personalized imaging outputs that are consistent with both the obtained scintigraphy images and the learned 3D activity distribution map.

In a first preferred embodiment of the second aspect of the present invention, the method further comprises a pre-training of the model comprising the steps of:
i. Providing a generic dataset comprising 3D anatomical and/or radionuclide distribution maps of patients, for instance Positron Emission Tomography (PET), Single Photon Emission Tomopgraphy (SPECT) images, Computed Tomography (CT) and/or Magnetic Resonance (MR) images;
ii. Training the generative Al model using the generic dataset to learn to generate 3D activity distribution maps from one, advantageously two, 2D images.

By pre-training the Al model on a broad dataset of 3D images from various patients the model gains a comprehensive understanding of spatial relationships and imaging characteristics across a wide range of scenarios. This foundational training equips the model with the ability to generate 3D activity distribution maps from one or two 2D images, providing a robust baseline for further refinement. The pre-training process ensures that the model is well-prepared to handle diverse imaging inputs and can effectively translate limited planar information into detailed 3D maps. This approach allows for quicker and more resource-effective imaging solutions as the patient-specific model does not need to be fully trained but only refined with the patient-specific data.

In another preferred embodiment of the second aspect of the present invention, training the model comprises the steps of:
I. Generating from a pre-radionuclide therapy PET or SPECT image of the patient a dataset of possible post-radionuclide therapy uptake distribution maps for the patient;
II. Generating a first dataset of synthetic planar scintigraphy images from the corresponding dataset of possible uptake distribution maps;
III. Training, using said first dataset of synthetic planar scintigraphy images and the corresponding possible uptake distribution maps for the patient as ground truth, a Convolutional Neural Network (CNN) generative machine learning model to generate synthetic 3D activity distribution maps from one, advantageously two, planar scintigraphy images.

By creating a first dataset of synthetic planar scintigraphy images and using the corresponding 3D PET or SPECT images as ground truth, the CNN model is trained to effectively translate limited planar information into detailed 3D activity maps. This training process ensures that the model captures the complex spatial relationships and patterns inherent in the 3D images, allowing it to generate synthetic maps that closely resemble actual 3D distributions. By using a patient-specific dataset derived from a pre-radionuclide therapy image this allows to produce personalized and clinically relevant outputs. By generating possible post-radionuclide therapy uptake distribution maps and corresponding synthetic planar images, the model is fine-tuned to accurately reflect individual patient anatomy and radionuclide distribution. This embodiment ensures that the synthetic maps are both accurate and tailored to the patient's unique physiological characteristics.

In a further preferred embodiment of the second aspect of the present invention, pre-training the model comprises the steps of:
i. Generating a second dataset of synthetic planar scintigraphy images from a corresponding generic dataset of 3D Positron Emission Tomography (PET) or Single Photon Emission Tomography (SPECT) images of patients;
ii. Training, using said second dataset of synthetic planar scintigraphy images and the corresponding 3D PET or SPECT images as ground truth, the Convolutional Neural Network (CNN) generative machine learning model to generate synthetic 3D activity distribution maps from one, advantageously two, planar scintigraphy images.

The use of a generic dataset for pre-training provides a comprehensive foundation for the model, enabling it to handle diverse imaging inputs and scenarios with improved efficiency and speed as the patient-specific CNN does not need to be fully trained but only refined with the patient-specific data.

In another preferred embodiment of the second aspect of the present invention, the method comprises adding noise to the generated synthetic planar scintigraphy images prior to training. By introducing noise into the synthetic images, the model is exposed to a wider range of data variability during the training process, which helps it learn to handle imperfections and uncertainties that may be present in real-world imaging scenarios. This exposure to noisy data improves the model's ability to generate accurate and reliable synthetic three-dimensional activity distribution maps even when the input images are less than ideal. Consequently, the model becomes more adept at producing clinically relevant outputs that are consistent with actual imaging conditions, thereby enhancing the precision of dosimetry evaluations and diagnostic assessments. Other sources of bias in the data such as scattered photons can also be added into the synthetic planar simulation to even further enhance the direct translation to real images.

In a further preferred embodiment of the second aspect of the present invention, the generative machine learning model is based on Diffusion Models (DM). By employing Diffusion Models, the method ensures robust generation of 3D maps that accurately reflect the complex spatial relationships and patterns inherent in nuclear medicine imaging data. This approach enhances the fidelity of the synthetic maps, improving dosimetry evaluations and diagnostic assessments. Consequently, the embodiment facilitates more precise treatment planning and monitoring in nuclear medicine, ultimately leading to better patient outcomes by ensuring that the synthetic maps are both accurate and clinically relevant. The use of Diffusion Models allows the generative Al model to produce reliable imaging outputs even in challenging scenarios, thereby expanding the applicability and utility of the invention across diverse clinical settings.

According to a third aspect, the present invention relates to a data processing system comprising means for carrying out the steps of any one of the methods of the present invention.

According to a fourth aspect, the present invention relates to a computer program product comprising instructions which, when the program is executed by a computer, cause the computer to carry out the steps of any one of the methods of the present invention.

According to a fifth aspect, the present invention relates to a computer-readable storage medium comprising instructions which, when executed by a computer, cause the computer to carry out the steps of any one of the methods of the present invention.

### Brief description of the drawings

- Figure 1 shows the workflow of a preferred embodiment of the method according to the first aspect of the present invention;
- Figure 2 shows the workflow of a preferred embodiment of the method according to the second aspect of the present invention;
- Figure 3 shows two specific implementations of the methods of the present invention used to generate the presented results;
- Figure 4 illustrate the refinement of the trained model;
- Figure 5 presents results using the different methodologies described. Axial (top), coronal (middle) and sagittal (bottom) views for an example patient are shown comparing the performance of baseline training.
- Figure 6 shows the average uptake relative to the mean value in the body for different organs measured from estimations using different methods; and
- Figure 7 presents (Top) an example of 3D distribution generation from measured ¹⁷⁷Lu-PSMA planar images in a prostate cancer patient. (Bottom) An example of 3D distribution generation from simulated planar images obtained from a post-therapy ¹⁸F-PSMA PET/CT of the same patient.

### Detailed description of preferred embodiments and presentation of experimental results

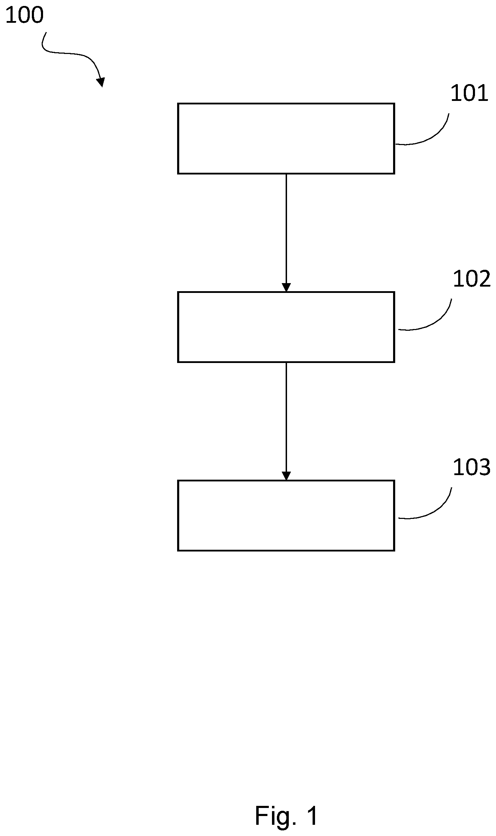

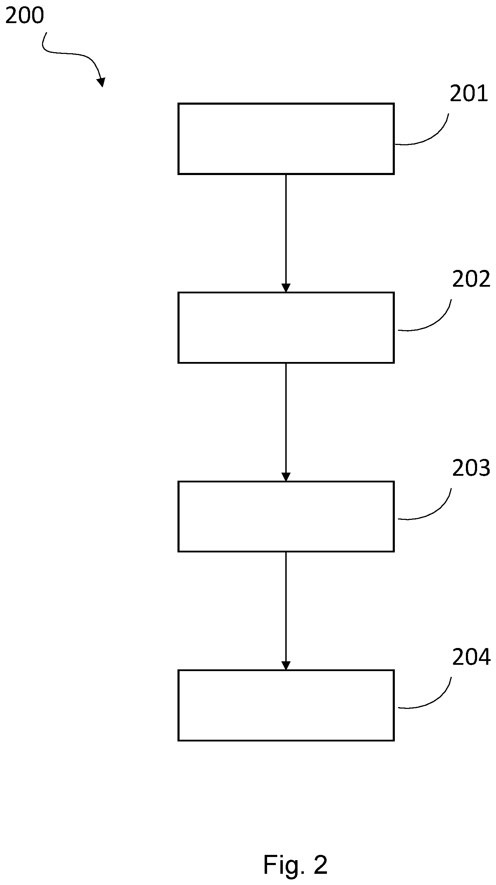

Preferred embodiments of the method according to the first aspect and the second aspect of the present invention are presented in Figures 1 and 2 and results as well as details about the implementation of the methods are presented in the other figures.

Figure 1 illustrates the workflow of a preferred embodiment 100 of the method for generating a synthetic post-radionuclide therapy three-dimensional (3D) activity distribution map of a patient. Method 100 comprises a first step 101, where at least one, advantageously two, post-radionuclide therapy planar scintigraphy images of the patient are obtained. Preferably, exactly two post-radionuclide therapy planar scintigraphy images of the patient are obtained, wherein these images are one frontal and one from the back of the patient, which can be obtained simultaneously with the use of dual head gamma cameras or sequentially at two different positions.

In step 102, a generative Artificial Intelligence (AI) model trained on a generic, i.e. not-patient specific, 3D medical imaging dataset , for example a dataset of Positron Emission Tomography (PET) or Single Photon Emission Computed Tomography (SPECT) images, to generate from one, advantageously two, planar images a synthetic 3D activity distribution map of the radionuclide is provided, wherein the model was further refined on a patient-specific dataset of simulated possible 3D uptake distribution maps of the radionuclide derived from a pre-radionuclide therapy 3D medical image, advantageously a nuclear medicine image, in particular a PET or SPECT image, of the patient. It is important to note that the pre-radionuclide therapy 3D medical image of the patient can also be a CT, an MRI or an Ultrasound image.

In step 103, the obtained planar scintigraphy images are employed as input or sampling constraint for said generative Al model to generate a synthetic post-radionuclide therapy 3D activity distribution map of the patient.

Figure 2 illustrates the workflow of a preferred embodiment 200 of the method for training a generative Al model to generate synthetic post-radionuclide therapy three-dimensional (3D) activity distribution maps of a patient. Method 200 comprises a first step 201 where a generic dataset, i.e. not patient-specific, comprising 3D anatomical and/or radionuclide distribution maps of patients, for instance Positron Emission Tomography (PET), Single Photon Emission Tomopgraphy (SPECT) images, Computed Tomography (CT) and/or Magnetic Resonance (MR) images of patients, is provided.

In step 202, the generative Al model is trained using the generic dataset to learn to generate 3D activity distribution map from one, advantageously two, 2D images.

In step 203, a patient-specific dataset of images derived from at least one pre-radionuclide therapy 3D anatomical and/or radionuclide image of the patient, for instance a Positron Emission Tomography (PET), Single Photon Emission Tomography (SPECT), Computed Tomography (CT) and/or Magnetic Resonance (MR) image, representing possible post-radionuclide therapy uptake distribution maps is provided.

Finally in step 204, the model is refined using the patient-specific dataset, such that the trained model is configured to generate a synthetic post-radionuclide therapy 3D activity distribution map of the patient using one, advantageously two, post-radionuclide therapy planar scintigraphy images as inputs or sampling constraints.

While the embodiments of Figures 1 and 2 comprise the use of a pre-trained model, or the pre-training of the model respectively, it is important to note that the methods of the present inventions are not limited to such embodiments. The methods of the present invention can be trained on a patient-specific dataset only and use such a model trained on this dataset instead of having a model pre-trained on a generic dataset.

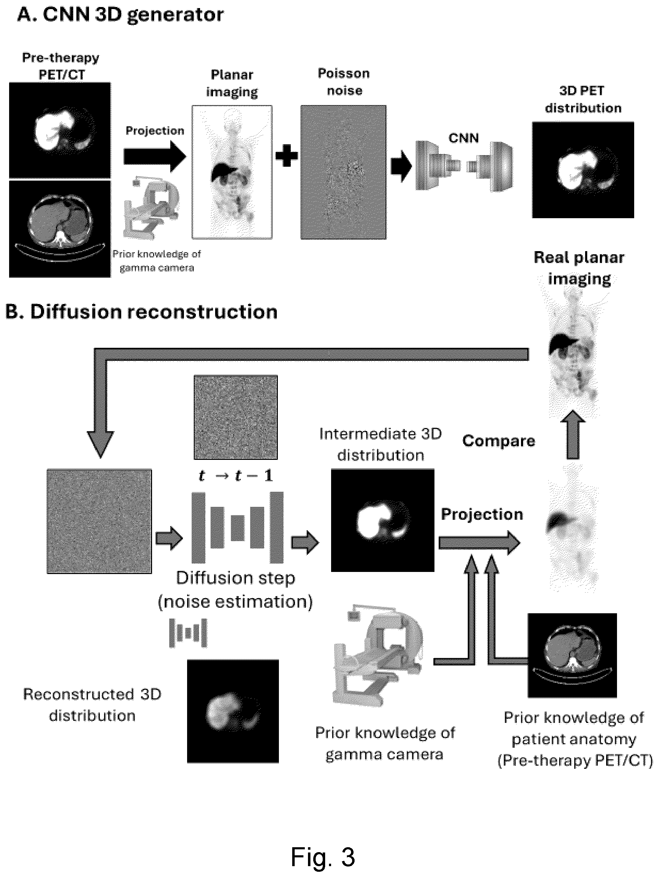

Figure 3 presents two possible specific implementations for the training method 200 using (A) a CNN model and (B) a Diffusion model as generative Al model on a generic dataset of 3D PET images.

In variant A, a first dataset of synthetic planar scintigraphy images from a corresponding dataset of 3D Positron Emission Tomography (PET) images is generated (see below for more details). A CNN model is then trained on this first dataset and the corresponding 3D PET as ground truth to generate synthetic 3D activity distribution maps from one, advantageously two, planar scintigraphy images.

The corresponding results presented below were generated by using a 3D ResUnet architecture described in (Lee et al., 2017) using paired 3D PET distributions and planar images via supervised learning. The input and output sizes were 128x128x175 voxels. The voxels were 5.7 mm size (isotropic). For the input, synthetic planar scintigraphy images (128x2x175) obtained according to the methodology described below from (128x128x175) 3D PET/CT images were used, the latter being used as reference images. The inventors used I_1 as the metric for the loss function and a total of 3000 epochs using Adam optimizer and a learning rate of 1·10⁻⁴. To make the generation of images robust to noise in the projections, the inventors added Poisson noise with different random magnitudes during training in the planar images prior to the input in the network. With this approach, the network is effectively learning the connection between the Poisson distribution *P*(*λ = A̅_{sc}*(*x, z*)) and the 3D PET images. This concept is based on the principles of variational autoencoders (VAE) where the encoded latent representation is often represented as a gaussian-like distribution instead of as a constant image which has been shown to produce better results than deterministic autoencoders. In the present case, the encoded operation is replaced by the analytical projection of the 3D PET images (known the attenuation from the CT) and the equivalent to the decoding operation is the 3D ResUnet.

In variant B, the generative Al model is a Diffusion Model (Diffusion MBIR) (Chung et al., 2023) that is trained to generate from a fully noisy 2D image a synthetic 3D activity distribution map. The network is specifically trained to learn the noise pattern for each time step given the solution of the previous time step. Specifically, a score based generative network (via diffusion models) following (Song et al., 2020) was trained to create synthetic axial slices of activity distributions from pre-radionuclide therapy dataset of ¹⁸F-PSMA patients. Diffusion models are based on the generation of images from pure noise (gaussian) initializations via several hundreds or thousands of time steps during which noise is gradually removed (1000-time steps used in this work). In diffusion models, the network is trained to learn the noise pattern for each time step given the solution of the previous time step. To generate 3D volumes from 2D synthetic slices matching the planar scintigraphy images, the inventors used the methodology described in (Lopez-Montes et al., 2024) including data consistency enforcement in every time step of the diffusion (enforcing the solution to be consistent with the planar distribution) and adding a smoothness penalty between consecutive axial views (Chung et al., 2023) to ensure consistent volume generation (preventing inter-slice artifacts since 3D volumes are created stacking consecutive 2D axial slices). It is important to notice that, in this approach, differently to method A, the training is performed without considering the planar images. The information of the planar and the enforcing of the matching between the generated set of slices and the acquired scintigraphy planar images is achieved during sampling. There, the process comprises guiding the model such that two-dimensional (2D) projection images generated from the synthetic 3D activity distribution maps match the actual post-radionuclide therapy planar scintigraphy images of the patient. This guiding comprises in particular computing 2D projections from the intermediate 3D activity distribution maps generated during sampling and enforcing consistency between the 2D projections and the obtained post-radionuclide therapy planar scintigraphy images using a loss function, thereby steering the sampling process towards generating a 3D activity distribution map that is consistent with both the obtained scintigraphy image and the learned 3D activity distribution map.

Both variants A and B involve computing synthetic planar scintigraphy images from 3D images. A 2D scintigraphy projection (*A_{sc}*) can be, for instance, obtained knowing the 3D distribution of activity (*A*_{3*D*}) in a patient and the attenuation of x-rays inside the body (*µ*) from the line integrals: ${A}_{sc} \left(u, v\right) = \int {A}_{3 D} \left(x, y, z\right) ⋅ {e}^{- {\int }_{0}^{s} μ \left(x′,y′,z′\right) ds ′} ds + r \left(u, v\right)$ (1)

Where *s* is line connecting every voxel of the volume (*x, y,* z) with every pixel of the acquisition plane (*u, v*)*. r*(*u, v*) includes other effects such as scattered photons and noise, generally Poisson like. The term in the exponential represents the integral of the total attenuation in the line (*s*) from the voxel (*x, y, z*) to the pixel (*u, v*)*.* Here, the commonly used approach in SPECT simulation of approximating equation (1) to a combination of a parallel projection with a PSF (gaussian) blurring factor given by the collimator properties of the acquisition camera and distance from the object to each plane *y* is applied : $\begin{matrix}{A}_{sc} \left(u, v\right) = {A}_{sc} \left(x, z\right) \approx \\ \int G \left(x, y, z\right) ∗ {A}_{3 D} \left(x, y, z\right) ⋅ {e}^{- {\int }_{0}^{y} μ \left(x′,y′,z′\right) dy ′} dy + r \left(x, z\right)\end{matrix}$ (2)

Scattered photons are here not considered. *r*(*x, z*) is modelled as Poisson noise obtained from the distribution *P*(*λ = A̅_{sc}*(*x, z*)) after scaling the projections to the expected number of detected counts (different number of counts were studied).

Common practice in scintigraphy is to obtain two images, one frontal and one from the back of the patient, which can be obtained simultaneously with the use of dual head gamma cameras or sequentially at two different positions. Here, it will be assumed simultaneously obtained frontal and back planar images of the patient. The inverse problem for equation (1) is generally regarded as unachievable with only two planar acquisitions. As it will be demonstrated below, by using previous information about the anatomy of the patient and expected post-radionuclide therapy uptake distribution maps, a post-radionuclide therapy 3D activity distribution map can be generated from one, preferably two, planar scintigraphy images using Al techniques.

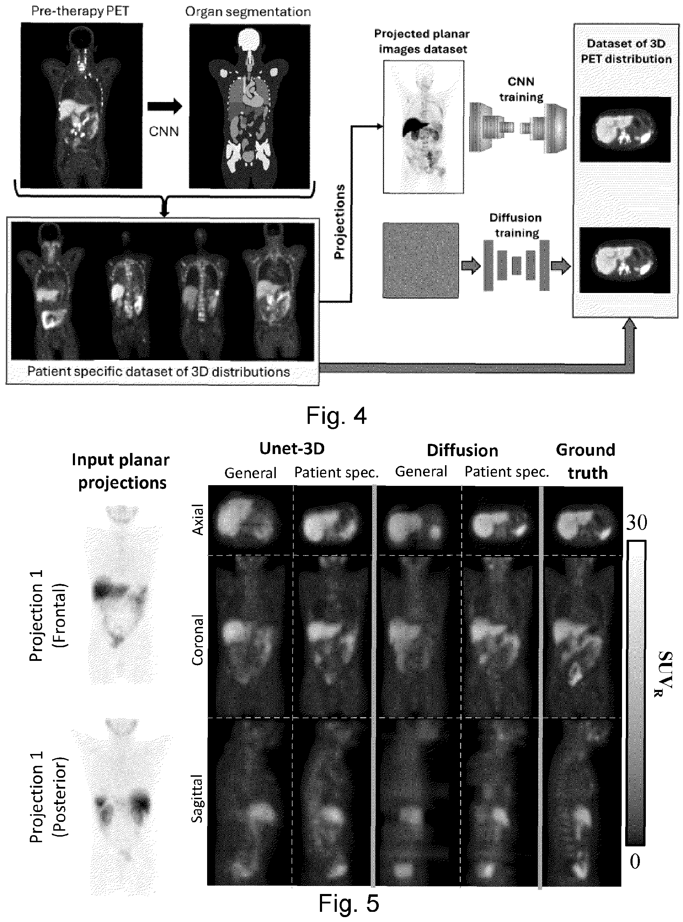

As mentioned above, in both the specific variants A and B, the model is trained on a generic. i.e. not patient-specific, dataset of 3D PET images. In order to ensure that the generated synthetic 3D activity distribution maps are precise, accounting for the anatomy and biology of the patient, the models were further refined according to the available information of the patient. For this purpose, the inventors used the pre-treatment PET/CT of the patient to generate a series of random realizations of possible uptakes for that specific patient. For that, the inventors used the CT-free PET based organ segmentation described in (Clement et al., 2024). They then generated 100 new distributions for every patient with variations in the uptake of every organ, and random rotations and translations of the patient. The new uptake for every organ was sampled from a uniform distribution from half to double the original activity in the organ. The inventors allowed random translations of the patient with an amplitude of 20 cm in each direction and random rotations of ± 5° in each 3D axis. From the possible uptake patterns, a second dataset of synthetic planar scintigraphy images is generated (with the method detailed above). The second dataset of synthetic planar scintigraphy images is then used as input and the corresponding possible uptake distribution maps for the patient as ground truth for refining the CNN model (variant A). The refinement of the DM used the possible uptake patterns as input and the second dataset of synthetic planar scintigraphy images as sampling constraints (variant B). The refinement of the models is present in Figure 4.

For demonstrating the feasibility of the methods of the present invention, the inventors used for the training of the CNN model and of the DM model a total of 46 long axial field-of-view (LAFOV) PET ¹⁸F-PSMA images obtained on a Biograph Vision Quadra (Siemens Healthineers) scanner. Planar images for training were obtained by simulation using the method above. For the validation, a dataset of 5 patients with pre- and post-radionuclide therapy PET/CT to generate synthetic planar images of the expected distribution after treatment were used. The pre-radionuclide therapy PET is used for the training of the refined model and the post-radionuclide therapy PET is used to simulate the planar scintigraphy images for validation. With this methodology, the ground truth 3D distribution (post-radionuclide therapy PET) is known to evaluate the accuracy of the different methods. For the quantification, the average activity predicted in the main relevant organs for PSMA: liver, kidneys, salivary glands and prostate was computed. All the synthetic planar data is simulated considering a parallel-hole collimator with a diameter of 1.5 mm and a length of 35 mm and a distance from the center of the patient to the detector of 500 mm. The inventors validated their method as well on real acquisitions from planar images. Note that for those cases the ground truth is not known, however, a comparison with an example with simulated planar images from a post-radionuclide therapy PET/CT compared with a real planar acquisition of the same patient is presented here.

Figure 5 shows 3D PET estimates from 2D planar scintigraphy following the different approaches described above. Unet-3D and Diffusion MBIR with patient-specific model refinement show more accurate representation of the ground truth 3D distribution compared to the case using generic 3D training, where the recovered organ and body shape produce relevant mismatches with respect to the baseline ground truth anatomy. In general, Diffusion MBIR showed a closer match with respect to the ground truth with an accurate recovery of all features in the plane of detector (*xz* plane), the information in the *y* direction, challenged by the collapse of the projections, appear more challenging with some features displaced in that direction from their real position. As global metrics, the mean absolute error (MAE) for the different methods with respect to the ground truth were for the case of the ResUnet model 0.76 for the generic learning, reduced to 0.56 for the patient specific training, while for the case of diffusion reconstruction, the generic training yielded a MAE of 0.68 and 0.53 for the patient specific training.

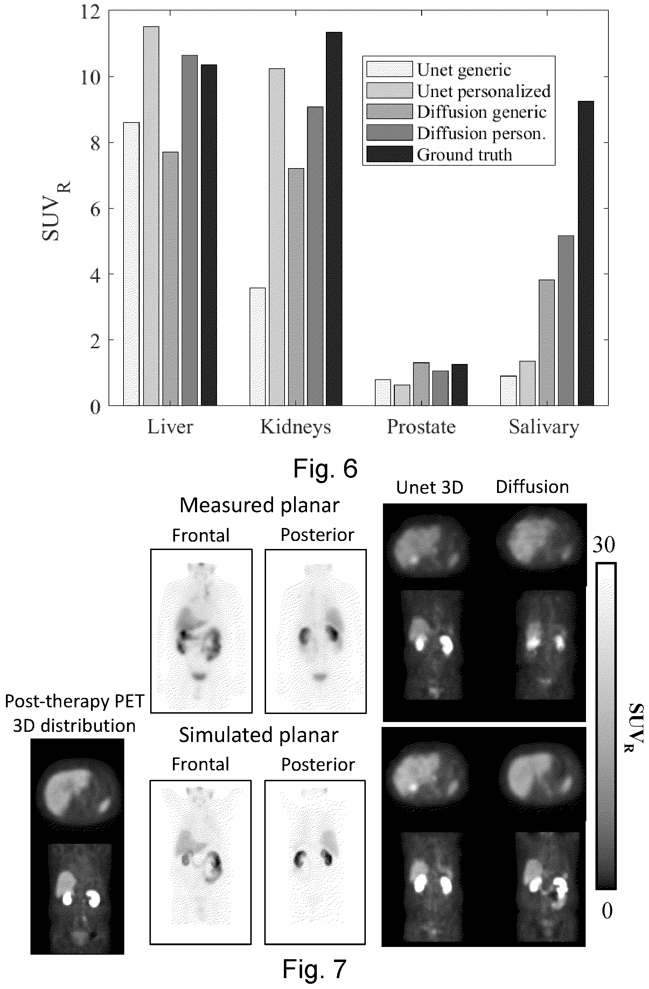

Figure 6 shows the quantification on different organs of the activity compared to the ground truth activity measured in the post-therapy PET/CT from which the planar images were obtained. Good recovery with relative errors < 10% for liver and <20% for kidneys for both patient specific training approach is observed while the mismatch was >30% for the generic training cases.

The performance of the method when using real measured planar images is shown in Figure 7. While for the case of measured planar images the ground truth 3D distribution is not available, a patient was selected for which a post-radionuclide therapy PET/CT at a different time point was also obtained. Thus, Figure 6 present the comparison of the results obtained from simulated planar images obtained from the post-therapy PET/CT and from measured planar images acquired in an actual gamma camera. Results in Figure 7 illustrate the accuracy of the simulated planar images when compared to real acquisitions and the consistency of 3D results when using measured planar images instead of simulations.

Important to note, that while the results above demonstrate the efficiency of the method when using 3D PET imaging, it is possible to train the models to replicate pseudo 3D SPECT images instead of 3D PET images by utilizing SPECT dataset instead of PET datasets in the training. However, by using PET-like images for the training instead of SPECT, it is possible to leverage extensive datasets of PET/CT images used for diagnostic and the use of the specific patient scans for the identification of prostate cancer malignancies and their metastasis. Also, the quality of PET images (improved resolution and signal to noise ratio) is generally superior compared to SPECT images.

While the ResUNet utilized in this work is trained to generate 3D volumes, the diffusion models presented here were trained for 2D slices and 3D volumes were generated by consecutive stack of slices including a TV regularization in the inter-slice direction to ensure consistent results. It is however possible to use 3D diffusion models that have experienced advances and with the use of last generation GPU's.

While the feasibility of the present approach focused on ¹⁷⁷Lu-PSMA has been show, it can be easily expanded to other nuclear agents with application in theranostics such as ¹²¹I for thyroid cancer, ²²³Ra for metastatic bone cancer and potentially many other radioligands which are currently under clinical trial.

### References

Chung, H., Ryu, D., Mccann, M. T., Klasky, M. L., & Ye, J. C. (2023). Solving 3D Inverse Problems using Pre-trained 2D Diffusion Models. IEEE/CVF Conference on Computer Vision and Pattern Recognition (CVPR). https://github.com/HJ-harry/DiffusionMBIR
Clement, C., Xue, S., Zhou, X., Li, L., Seifert, R., Rominger, A., Liu, J., & Shi, K. (2024). Multi-Organ Segmentation on CT-free Total-Body Dynamic PET Scans. Journal of Nuclear Medicine, 65(supplement 2), 241953. http://jnm.snmjournals.org/content/65/supplement_2/241953.abstract
Lee, K., Zung, J., Li, P., Jain, V., & Seung, H. S. (2017). Superhuman Accuracy on the SNEMI3D Connectomics Challenge. http://arxiv.org/abs/1706.00120
Lopez-Montes, A., Mcskimming, T., Skeats, A., Delnooz, C., Gonzales, B., Zbijewski, W., & Sisniega, A. (2024). Stationary CT Imaging of Intracranial Hemorrhage with Diffusion Posterior Sampling Reconstruction.

## Claims

1. A computer-implemented method for generating a synthetic post-radionuclide therapy three-dimensional (3D) activity distribution map of a patient comprising:
a. Obtaining at least one, advantageously two, post-radionuclide therapy planar scintigraphy images of the patient;
b. Providing a generative Artificial Intelligence (AI) model trained on a patient-specific 3D medical imaging dataset of radiotracer distribution derived from at least one pre-therapy image of the patient, preferably from a nuclear medicine technique such as a Positron Emission Tomography (PET) or Single Photon Computed Tomography (SPECT), or alternatively derived from an anatomic image technique such as Computed Tomography (CT), to generate from one, advantageously two, planar images a synthetic 3D activity distribution map of the radionuclide;
c. Employing the obtained planar scintigraphy images as input or sampling constraint for said generative Al model to generate a synthetic post-radionuclide therapy 3D activity distribution map of the patient.

2. The method according to claim 1, wherein the patient-specific 3D medical imaging dataset of radiotracer distribution comprises or consists of simulated possible 3D uptake distribution maps of the radionuclide derived from the at least one pre-radionuclide therapy 3D medical image.

3. The method according to any one of the claims 1 or 2, wherein the model has been pre-trained on generic population datasets to generate 3D activity distributions from 2D planar images.

4. The method according to any one of the preceding claims, wherein knowledge on the acquisition instrument and/or parameters used to acquire the planar scintigraphy images is included in the training, the refining and/or sampling of the model.

5. The method according to claim 4, wherein the knowledge on the acquisition instrument and/or parameters comprises the type of radionuclide, the energy of emitted photons, the collimator type and properties, septa size, type of detector, pixel size of the detector, the energy window settings, the acquisition time and the distance between the detector and the patient.

6. The method according to any one of the claims 1 to 5, wherein anatomical and/or radionuclide distribution information corresponding to the 3D nuclear medical images, preferably PET/CT or SPECT/CT images, is included in the training and/or sampling of the model.

7. The method according to claim 6, wherein the anatomical information is obtained from Computed Tomography (CT), Ultrasounds (US) or Magnetic Resonance (MR) images scans.

8. The method according to any one of the preceding claims, wherein the generative machine learning model is a Convolutional Neural Network (CNN) model and said CNN model has been trained using a dataset comprising synthetic planar scintigraphy images derived from 3D PET images and the corresponding original 3D PET images serving as ground truth for training, such that the trained CNN model generates a 3D activity distribution map from one, advantageously two, input planar scintigraphy images.

9. The method according to any one of the claims 1 to 7, wherein the generative machine learning model is based on Diffusion Model (DM) and the sampling process comprises guiding the model such that two-dimensional (2D) projection images generated from the 3D activity distribution map match the obtained post-radionuclide therapy planar scintigraphy image of the patient.

10. The method according to claim 9, wherein guiding the DM comprises:
i. computing 2D projections from the intermediate 3D activity distribution maps generated during sampling and the anatomical information of the patient; and
ii. enforcing consistency between said 2D projections and the obtained post-radionuclide therapy planar scintigraphy images using a loss function, thereby steering the sampling process towards generating a 3D activity distribution map that is consistent with both the obtained scintigraphy images and the learned 3D activity distribution map.

11. A computer-implemented method for training a generative Al model to generate synthetic post-radionuclide therapy three-dimensional (3D) activity distribution maps of a patient, comprising:
a. Providing a patient-specific 3D medical imaging dataset of radiotracer distribution derived from at least one pre-radionuclide therapy 3D anatomical and/or radionuclide image of the patient, for instance a Positron Emission Tomography (PET), Single Photon Emission Tomography (SPECT), Computed Tomography (CT) and/or Magnetic Resonance (MR) image, representing possible post-radionuclide therapy uptake distribution maps;
b. Training the generative Al model using the patient-specific dataset to learn to generate 3D activity distribution maps from one, advantageously two, 2D images, such that the trained model is configured to generate a synthetic post-radionuclide therapy 3D activity distribution map of the patient using one, advantageously two, post-radionuclide therapy planar scintigraphy images as inputs or sampling constraints.

12. The method according to claim 11 comprising a pre-training of the model comprising the steps of:
i. Providing a generic dataset comprising 3D anatomical and/or radionuclide distribution maps of patients, for instance Positron Emission Tomography (PET), Single Photon Emission Tomopgraphy (SPECT) images, Computed Tomography (CT) and/or Magnetic Resonance (MR) images;
ii. Training the generative Al model using the generic dataset to learn to generate 3D activity distribution maps from one, advantageously two, 2D images.

13. The method according to claim 11, wherein training the model comprising the steps of:
I. Generating from a pre-radionuclide therapy PET or SPECT image of the patient a dataset of possible post-radionuclide therapy uptake distribution maps for the patient;
II. Generating a first dataset of synthetic planar scintigraphy images from the corresponding dataset of possible uptake distribution maps;
III. Training, using said first dataset of synthetic planar scintigraphy images and the corresponding possible uptake distribution maps for the patient as ground truth, a Convolutional Neural Network (CNN) generative machine learning model to generate synthetic 3D activity distribution maps from one, advantageously two, planar scintigraphy images.

14. The method according to claims 11 to 13, wherein pre-training the model comprising the steps of:
i. Generating a second dataset of synthetic planar scintigraphy images from a corresponding generic dataset of 3D Positron Emission Tomography (PET) or Single Photon Emission Tomography (SPECT) images of patients;
ii. Training, using said second dataset of synthetic planar scintigraphy images and the corresponding 3D PET or SPECT images as ground truth, the Convolutional Neural Network (CNN) generative machine learning model to generate synthetic 3D activity distribution maps from one, advantageously two, planar scintigraphy images.

15. The method according to any one the claims 11 or 12, wherein the generative machine learning model is based on Diffusion Models (DM).
